# EUROPEAN PATENT APPLICATION

(11) **EP 1 471 735 A2**
(43) Date of publication of application: **27.10.2004**
(21) Application number: 04009127.4
(22) Date of filing: 16.04.2004
(51) Int. Cl.: H04N 5/445

(54) **Customized EPG display with visual cues**

(30) Priority: 23.04.2003 US 421072
(71) Applicant: MICROSOFT CORPORATION, Redmond, Washington 98052-6399 (US)
(72) Inventor: Wagner, Mark, Seattle, Washington 98112 (US)
(74) Representative: Grünecker, Kinkeldey, Stockmair & Schwanhäusser Anwaltssozietät

(57) **Abstract**

A TV viewer selects viewing interests and a corresponding highlighting from a user interface. An enhanced Electronic Programming Guide (EPG) display is output and includes TV shows, channels and time slots within a programming range. TV shows having characteristics that match the TV viewer's selected viewing interests are displayed in the enhanced EPG display with the corresponding highlighting.

## Description

### TECHNICAL FIELD

The present invention relates generally to schedules for programming information viewed upon a display screen. More specifically, the present invention relates to an electronic program guide that can be enhanced by a viewer-specified search with respect to the way that a schedule of programming information is presented to the viewer.

### BACKGROUND

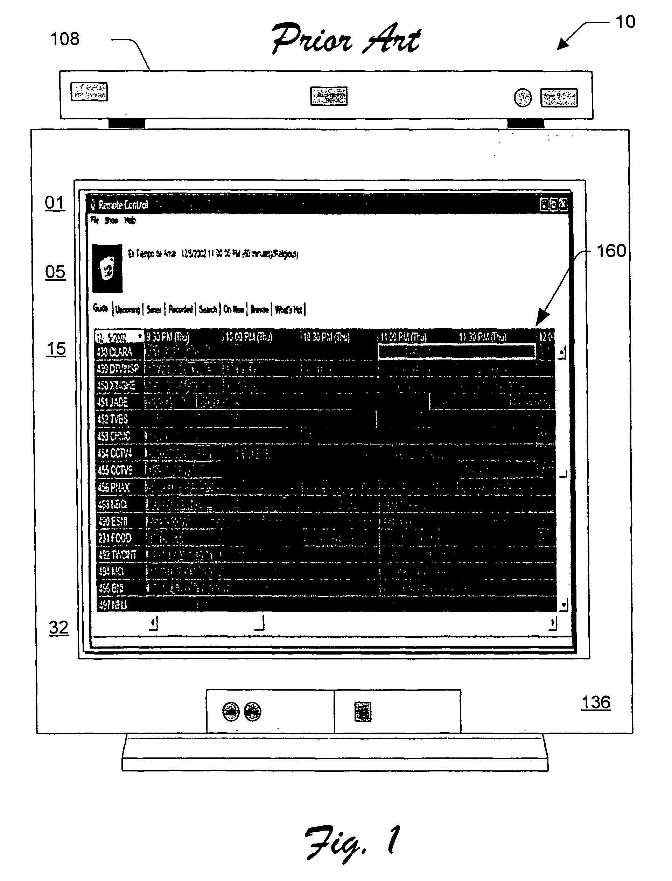

Electronic program guides (EPGs) enable TV viewers to navigate through an onscreen program guide and locate shows. The EPG enables a viewer to look at schedules of current and future programming, set reminders for upcoming programs, or enter instructions to record one or more shows. Fig. 1 shows an entertainment system 10 that includes a client device 108 and a display device or monitor such as a TV 136. Client device 108 receives a data for an EPG from a source such as a broadcast source. An EPG application is executed by client device 108 to produce output. The output is rendered at TV 136 as an EPG display 160. EPG display 160 has a number of horizontal positions represented in Fig. 1 by reference numerals 01, 05, 15, and 32. At horizontal position 15 and before horizontal position 32, a presentation is made of a programming lineup for channels 438 through 497 for the 9:30 PM until about midnight time slot. An indictor just prior to horizontal position 15 shows the date to be December 5, 2002. Horizontal position 15 has a TV show on the EPG display 160 highlighted for channel 438 at the 11:00 PM through midnight timeslot. This highlighted TV shows is further described from data in the EPG at horizontal position 05. Horizontal position 32 features conventional slider controls that allow a horizontal look at portions of the EPG display 160 prior to 9:30 PM and after midnight. Perpendicular to the slider controls of horizontal position 32 are conventional slider controls that allow a vertical look at portions of the EPG display 160 less than channel 438 and higher then channel 497. One of more input devices, discussed below, can be used to select a displayed TV show to view, to request more information about a displayed TV show, and to perform other conventional EPG functions.

Many cable and satellite television services offer dozens if not hundreds of different channels from which the viewer may choose. The dramatic increase in the amount of available broadcast programming and other services greatly increases the amount and type of available information accessible by the viewer. Unfortunately, the increase and quantity of information, e.g., broadcast programming and services, complicates the process of program and service selection. Unless the viewer is able to quickly and easily identify desired programs and services and determine when those programs and services are available, the viewer will not realize the full potential for using and accessing the available wealth of knowledge and entertainment.

Although advances in EPGs provide a viewer with flexibility in viewing broadcast programming information, a significant problem occurs due to way that programming information is presented to a viewer on a display screen. Specifically, the viewer must scroll through numerous broadcast programming and services in an EPG to find a broadcast program or service that the viewer is interested in viewing.

It would therefore be an advance to enhance the EPG presentation of programming information to assist a viewer in finding a broadcast program or service that the viewer is interested in viewing.

### SUMMARY

A TV viewer selects viewing interests and a highlighting corresponding thereto using a User Interface (UI). An EPG display reflecting the TV viewer's selections is displayed. Each TV show in the EPG having characteristics that match the selected viewing interests is displayed with the corresponding highlighting.

### BRIEF DESCRIPTION OF THE DRAWINGS

The Detailed Description is described with reference to the accompanying Figures in which the same numbers are used throughout the disclosure and the Figures to reference like components and features.

Fig. 1 illustrates, in a front elevation view, a conventional environment in which a client device outputs a portion of an electronic program guide (EPG) resulting in an EPG display upon a television (TV), where the EPG display lists each of a plurality TV programs scheduled for broadcast.

Fig. 2. illustrates an exemplary display including a user interface (UI) by which a viewer operates controls of the UI to select one or more viewing interests and a highlighting corresponding thereto for display on a resultant enhanced EPG display.

Fig. 3. illustrates the display of Fig. 2 after operation of the controls thereof by a viewer.

Fig. 4 illustrates, in a front elevation view, the client device of Fig. 1 having output a portion of a resultant enhanced EPG display to the television for optional selection by the viewer of the displayed programming.

Fig. 5 shows a flow chart illustrative of a process in which a viewer answers a questionnaire, an EPG is queried for programming matching the viewer's answers, and a resultant enhanced EPG display can be seen by the viewer for optional selection of the displayed programming.

Fig. 6 illustrates an exemplary system that provides a suitable operating environment in which the present invention can be either fully or partially implemented, and more particularly showing a content distribution system that is broadcasting to a plurality of client devices, where both the content distribution system and each client device are in communication with a two-way network.

Fig. 7 illustrates of an example client device, a television, and various input devices that interact with the client device.

Fig. 8 is a block diagram that illustrates components of the example client device(s) shown in Figs. 6-7.

### DETAILED DESCRIPTION

This invention allows a television (TV) viewer to select TV shows from an enhanced Electronic Programming Guide (EPG) display. The enhanced EPG display has visual cues that have been selected by the TV viewer. The visual cues indicate TV shows that have characteristics that match what the TV viewer's specified viewing interests. A User Interface (UI) allows the TV viewer to select both a viewing interest and a visual cue representing that interest. Data for the enhanced EPG display includes characteristics for each TV show. The data for the enhanced EPG that can be stored at the client device 108 or the data can be stored remotely.

The data in an electronic programming guide can include a plurality of TV shows for a plurality of channels over a period of time. The data also includes characteristics for each TV show. These characteristics can be represented by in metadata or in another type of data. The characteristics of the TV show can be quite diverse, such as a title of the TV show, a story line of the TV show, a description of the TV show, production credits for the TV show, a critic's opinion of the TV show, a review for the TV show, a recommendation of the TV show, a program length range of the TV show, a commercial-free indicator for the TV show, a genre of the TV show, a rating for the TV show, a performer in the TV show, a director of the TV show, an award won by the TV show, a date relevant to the TV show, and other characteristics that represent facts specific to the TV show.

The characteristics of each TV show for the enhanced EPG display processed by a comparison routine to find matches with the interests that the TV viewer has previously selected using the UI. When a matching TV show is displayed on the enhanced EPG display, the matching TV show will be displayed with the corresponding selected visual cue. Accordingly, TV shows having characteristics that match selected viewing interests are displayed in the enhanced EPG display with the corresponding highlighting selected by the TV viewer.

Referring now to Figs. 1-2, a User Interface (UI) 200 can be displayed upon the TV 136. UI 200 presents a questionnaire to a TV viewer. The client device 108 can execute an enhanced EPG application to produce output. The output is rendered at TV 136 as the UI 200. UI 200 presents a questionnaire to a TV viewer. The TV viewer uses an input device to interface with UI 200 so as to specify interests in TV viewing as answers to the questionnaire. The enhanced EPG application has access to electronic programming guide data for the TV shows. The enhanced EPG application also has access to the TV viewer's selections that are to be indicated by the TV viewer's use of, and input to, UI 200.

UI 200 has a plurality of triplet controls that can be used by the TV viewer to complete the questionnaire. The triple controls include a category control 210, an identifier control 212, and a visual cue control 214. A pull down menu control 202 is associated with each of the controls 210, 212, and 214 which, when activated, responds with a display of a menu from which the TV view can make a selection. The displayed menu for the category control 210 includes a list of viewing interest categories. The displayed menu for the identifier control 210 includes a list of identifiers that are specific to the selected menu item for the category control 210. The displayed menu for the visual cue control 214 includes a list of visual cues. The TV viewer makes a selection from among the list of visual cues. As such, the selected visual cue will be seen by the TV viewer on the enhanced EPG display when a TV show is displayed that matches both the selected menu item for the category control 210 and the selected menu item for the identifier control 212. Any of the lists that are displayed after activating a pull down menu function can be stored locally at, or remotely from, the client device 108.

As an alternative to the pull down menu for the identifier control 212, a TV viewer can use an input device to input a specific character string to the client device 108 for entry into the field of the identifier control 212. By way of example, if the TV viewer does not see what should be selected in the displayed list after activating the pull down menu function for the identifier control 212, then the TV viewer uses a remote control device to enter a character string, for example, the string "FOOD". As such, this character string will be matched with what has been specified for the category control 210 against the characteristics of TV shows in the data of an electronic programming guide. Accordingly, the TV viewer need not be limited with respect to the list of choices displayed by any of the pull down menus for any of the controls. For example, the TV viewer can use the input device to enter a character, character string, or icon into the field for visual cue control 214. Then, a triplet control that matches with a corresponding TV show in the data of the electronic programming guide will be displayed on the enhanced EPG display with the character, character string, or icon that was directly input by the TV viewer.

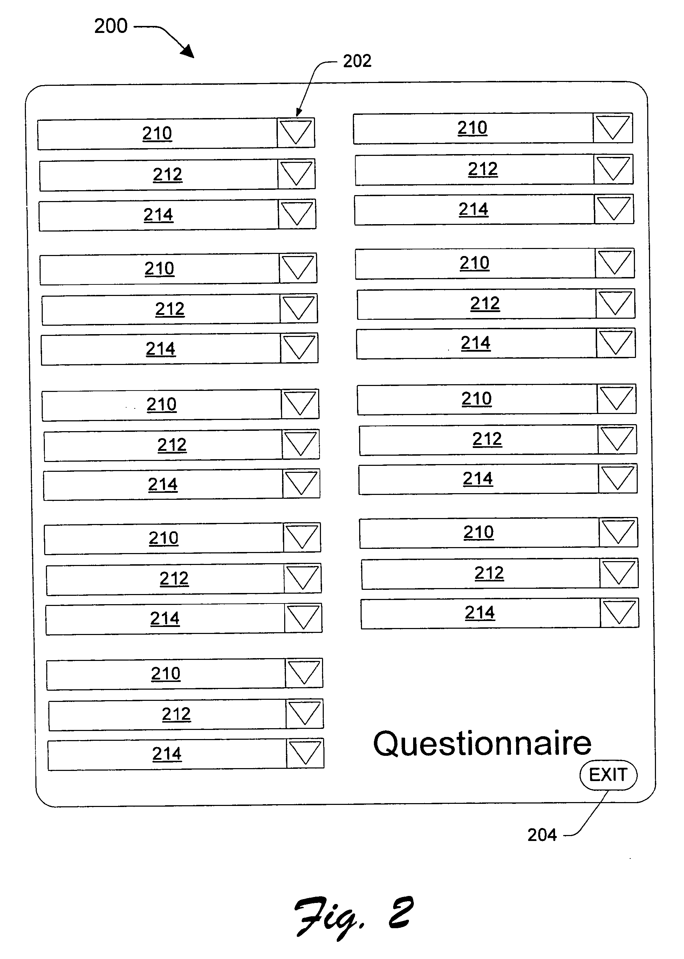

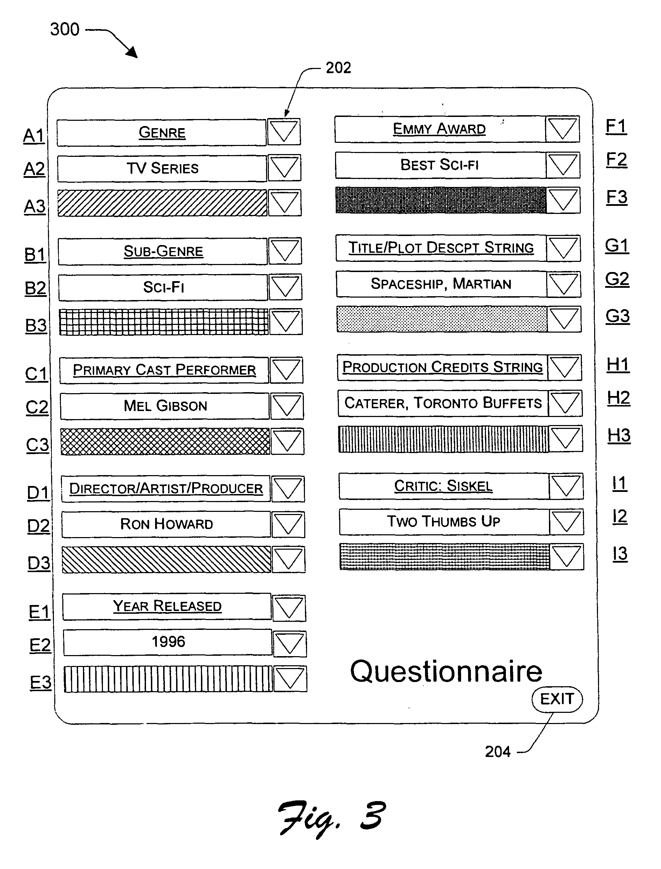

An exemplary UI 300 is seen in Fig. 3, where the TV viewer has completed making selections via use of the depicted controls. Although UI 300 shows that the TV view has made all of the selections that UI 300 allows, of course, the TV viewer can make less than all of the selections than are depicted in UI 300. Each triplet control in UI 300 has a number of horizontal positions represented in Fig. 3 by the reference alphanumerics of a letter followed by 1, 2, or 3. Here, the numbers 1-3 corresponds, respectively, to the category control 210, the identifier control 212, and the visual cue control 214 seen in Fig 2.

Fig. 3 shows that a different filling pattern has been selected by the TV viewer for each of the visual cue controls A3-I3. It is intended that the TV viewer can select visual cue other than filling patterns from the menu when pull down menu control 202 is activated for visual cue controls A3-I3. The visual cues that can be selected by the TV viewer from the menu include, but are not limited to, a text appearance, a border appearance, a background appearance, shading within a border, a text color, an alignment of text, a border appearance, a background color, a shading, an icon, a typeface, a font, a font style, a font size, a text effect, and combinations thereof.

After a TV viewer has input all of their selections into the user interface, such as is seen in UI 300, the TV viewer activates an exit control 204. When the exit control 204 is activated, a phase of the enhanced EPG application begins. In this phase, the enhanced EPG application is executed by client device 108 such that the characteristics of TV shows in the data of the electronic programming guide are compared to the selected category and identifier (210, 212) for each triplet control (A1-A2, B1-B2, C1-C2, D1-D2, E1-E2, F1-F2, G1-G2, H1-H2, and I1-I2). The comparisons by the enhanced EPG application are conducted for the purpose of finding matches between the TV viewer's selections displayed on the user interface and the characteristics of TV shows in the data of the electronic programming guide.

The comparisons can include an assessment of TV shows for which there is more than one such match. In these cases, a predetermined algorithm can be used to determine which of the selected visual cues is to be used to display each TV show for which there are multiple matches to its characteristics. For example, the predetermined algorithm can include a hierarchical schema of the importance of specific viewer interests. One such hierarchical schema can be incorporated into the enhanced EPG application and/or configuration of the client device 108. By way of example, significance can be attached to the letter in the reference alphanumerics (e.g., A, B, ..., I) seen in UI 300. By way of example and not by way of limitation, the lower that the letter is in the alphabet, the higher the assessment of the TV viewer's interest level. As such, the TV viewer can be deemed to be more interested in those viewing interests specified for letter 'A' for the triplet control 210, 212, 214 and is less interested in viewing interests specified for letters further down in the alphabet. Stated otherwise, the more important interests are in the higher and in the first column of triplet controls 210, 212, 214 in UI 200. Interests of low importance to the TV viewer are specified lower and in the second column of triplet controls 210, 212, 214 in UI 200.

The foregoing hierarchical understanding can be used to resolve questions. One such question is related to which visual cue should be used to show a TV viewer's interests in the enhanced EPG display when more than one characteristic for the TV show matches both the selected category and the identifier (210, 212) for more than one triplet control. For instance, the selected visual cue that corresponds to the lowest consecutive matching visual cue control (A3-I3) can be displayed for that TV show on the enhanced EPG display. By way of example using UI 300, if the electronic programming guide data contains a TV show having the characteristics of a TV series, a science fiction work, staring Mel Gibson, and released in 1996, then the TV show would be displayed in the enhanced EPG display with the E3 visual cue. As another example using UI 300, a TV show having the characteristics of a TV series, a science fiction work, and production credits listing "Caterer, Toronto Buffets" would be displayed in the enhanced EPG display with the B3 visual cue. Here, the H3 visual cue would not be used because a consecutive match did not exist between C1-C2 through G1-G2 and the characteristics of the TV show. As a further example using UI 300, a TV show having characteristics that only match the production credits listing "Caterer, Toronto Buffets" would be displayed in the enhanced EPG with the visual cue H3. Similarly, a TV show having the matching characteristics for UI 300 that are limited only to being a science fiction work (B2) for which the critic Siskel gave a rating of "Two Thumbs Up" (I2) would be displayed in the enhanced EPG with the visual cue B3. The visual cue B3 would be used because it was the hierarchically highest match after which there were no consecutive matches.

Still other implementations are contemplated that can be used to resolve questions related to which visual cue should be used to show a TV viewer's interests in the enhanced EPG display. For instance, whenever a characteristic of a TV show in the data of an electronic programming guide matches both the selected category and the identifier (210, 212) for a triplet control, the selected visual cue that corresponds to the matching visual cue control (A3-I3) would be preliminarily designated for display of that TV show on the enhanced EPG. Then, any other matches for that TV show would be handled by the rule hat the selected visual cue is used in the EPG display that corresponds to the lowest of the consecutive matches that begin at the match of A1-A3. As such, each and every matching TV show would receive one of the selected visual cues, and any TV show having multiple matches would be displayed with the visual cue of the hierarchically lowest consecutive match.

As mentioned above, the enhanced EPG application and the client device 108 can be configured in various ways with respect to the significance of the order or organization of the letter and reference alphanumerics (e.g., A1-3, B1-3, ..., I1-3). For instance, implementations can provide that more than one visual cue is displayed for one TV show on the enhanced EPG. Accordingly, the foregoing examples are not intended to limit the decision processes in which the enhanced EPG application and/or the client device 108 assigns visual cues (A3-I3) that are to appear on the enhanced EPG display.

The TV viewer selections indicted in UIs 200-300 are used by the client device 108 in the execution of the enhanced EPG application. The enhanced EPG application has access to data of an electronic programming guide. This data can be received from a source, such as a broadcast source, at the client device 108. Other sources of the data are also contemplated.

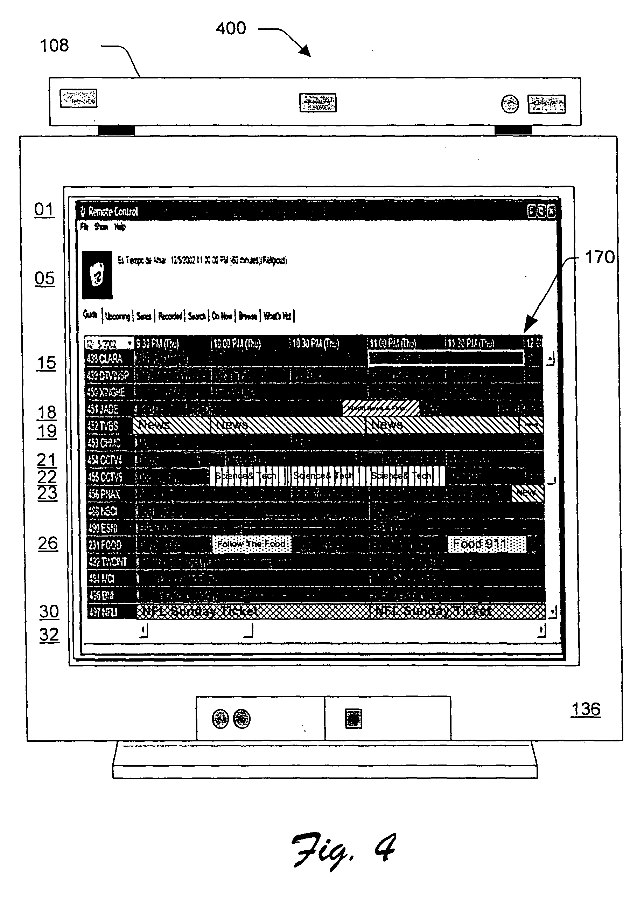

Fig. 4 shows an exemplary environment 400 where TV 136 displays an example of an enhanced EPG 170. Enhanced EPG 170 is displayed on TV 136 having been received for rendering from client device 108. The output is rendered at TV 136. Enhanced EPG 170 has a number of horizontal positions represented in Fig. 4 by reference numerals 01, 05, 15, 18, 19, 21-23, 26, and 32. A total of thirteen (13) visual cues are seen on the enhanced EPG 170. For the benefit of the reader, the specific examples given in Fig. 3 do not correspond to the thirteen (13) visual cues seen in Fig. 4. The thirteen (13) visual cues are as follows: the TV show "World News & Fins." at reference numeral 18, four (4) "News" TV shows at reference numeral 19, three (3) "Science and Tech" TV shows at reference numeral 22, one news program at reference numeral 23 at reference numeral 18, two (2) programs having the term "Food" in the visual cue at reference numeral 26, and two (2) "NFL Sunday Ticket" TV shows at reference numeral 30. Each visual cue represents a match between the controls 210-214 of the triplet A3-I3 that were specified on UI 200 and the corresponding characteristics of the TV show displayed on the enhanced EPG 170. For example, UI 200 may have been used by the TV viewer to specify for the category and identifier control (210, 212) as follows: (News, World News); (News, Local News); (TV Series, Science & Tech.); (Topic, Food); (Sports, Football Game). As such, the corresponding highlight or visual cue 214 for each of the foregoing is seen in the enhanced EPG 170.

Fig. 4 shows most of the TV shows in the enhanced EPG 170 are not emphasized with highlighting or visual cues because their respective characteristics did not match the TV viewer's specification. As an alternative, these TV shows can be grayed out and/or further deemphasized to reflect greater contrast in the enhanced EPG 170. It is further contemplated that highlighting and/or visual cues could be used to display TV shows in the EPG that did not match the TV viewer's specifications, while the display of the matching TV shows would not be emphasized. Variations and combinations of the foregoing are also contemplated.

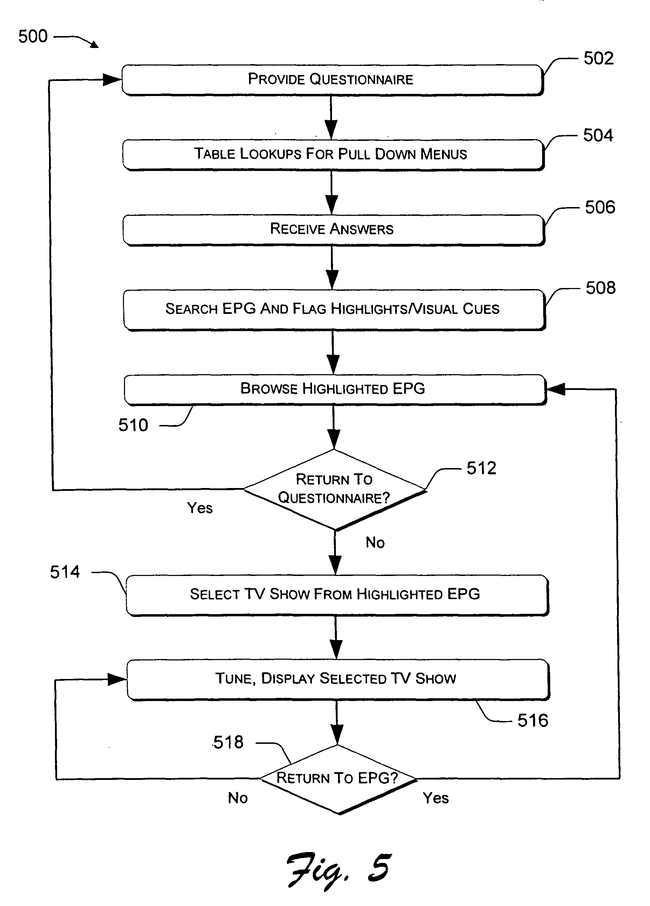

A process 500 is seen in Fig. 5 which can be used, for example, with respect to environment 400 depicted in Fig. 4. In reference to Figs. 2-4, process 500 begins at block 502 where a questionnaire is provided to a TV viewer via a user interface (UI). An example of the action taken at block 502 is seen in Fig. 2. After the TV viewer activates a pull down menu control 202, block 504 performs table lookups for the each pull down menu function. The table lookups are used to obtain a list to be displayed to the TV viewer. The TV viewer makes a selection from the displayed list. Alternatively, as discussed above, the TV viewer can use an input device to directly key-in or enter a selection in the form of a character, a character string, an icon, etc. Once the TV viewer has made all of the selections desired, such as is seen by UI 300 in Fig. 3, the selections are received for processing by an enhanced EPG application executing in client device 108.

At block 508, the data in an EPG stored in the client device 108 is searched with the enhanced EPG application. Matches are located and highlighting or visual cues for the matches that are to be displayed in the enhanced EPG are determined based upon predetermined criteria stored in the enhanced EPG application and/or the client device 108. The resultant enhanced EPG display is displayed at block 510, an example of which is seen in Fig. 4. The TV viewer can browse the enhanced EPG display with an input device to scroll the display using slider controls as previously discussed with respect to Fig. 1.

At block 512 of process 500, a query is made as to whether the TV viewer has input a command requesting a return to the questionnaire. If so, then process 500 moves to block 502. If this command has not been entered, then the TV viewer will have input a request at block 514 to view a TV show that is displayed on the enhanced EPG display. The requested TV show that is selected by the TV viewer on the enhanced EPG display need not be one that has a highlight or a visual cue associated with its appearance.

After block 514, process 500 moves to block 516 where a tuner in the client device 108 tunes to the channel of the corresponding requested TV show. The client device 108 outputs a display of the TV show for rendering on the TV 136. The TV viewer can then watch the requested TV show. Alternatively, when the TV viewer has input a designation of a TV show on the enhanced EPG display, the TV viewer can also input a request that the characteristics of the designated TV show are to be matched against the characteristics of other TV shows in the data of the electronic programming guide to find any matches. In this case, the enhanced EPG application can be configured to display those matching TV shows, with the highlighting corresponding to the requested TV show, in another enhanced EPG display. The TV viewer can then select the same or a different TV show on this enhanced EPG display.

After block 516, process 500 moves to block 518 where a query is made as to whether the TV viewer has input a'request to have the enhanced EPG displayed again. If so, then block 510 is performed again. Otherwise, process 500 loops between blocks 516 and 518 while waiting for a command from the TV viewer.

### Exemplary Environment

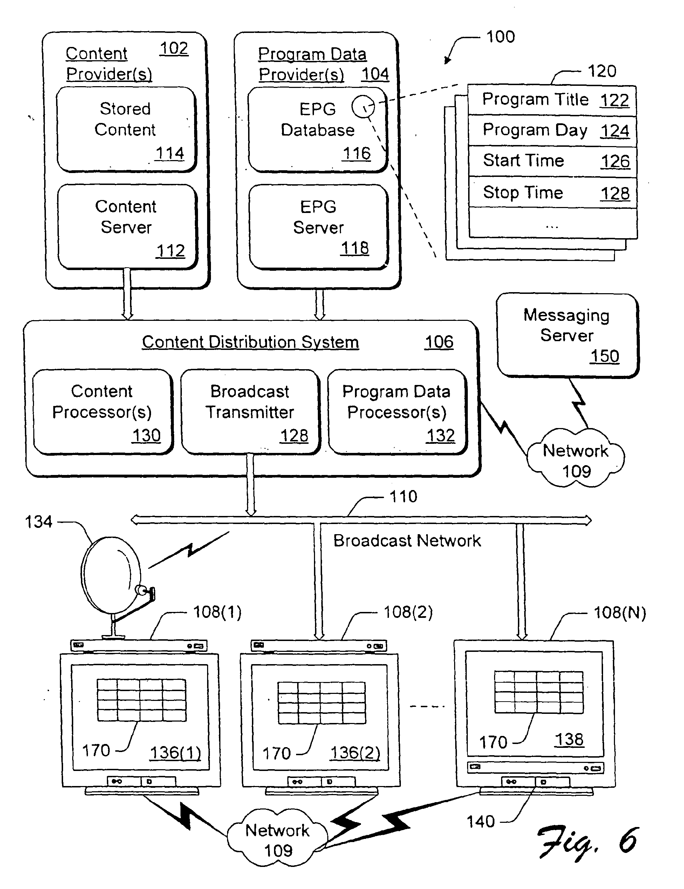

Fig. 6 illustrates an exemplary environment 100 in which the present invention can be fully or partially implemented. Exemplary environment 100 is a television entertainment system that facilitates distribution of content and program data to multiple viewers. The environment 100 includes one or more content providers 102, one or more program data providers 104, a content distribution system 106, and multiple client devices 108(1), 108(2), ..., 108(N) coupled to the content distribution system 106 via a broadcast network 110.

Content provider 102 includes a content server 112 and stored content 114, such as movies, television programs, commercials, music, and similar audio and/or video content. Content server 112 controls distribution of the stored content 114 from content provider 102 to the content distribution system 106. Additionally, content server 102 controls distribution of live content (e.g., content that was not previously stored, such as live feeds) and/or content stored at other locations to the content distribution system 106.

Program data provider 104 includes an electronic program guide (EPG) database 116 and an EPG server 118. The EPG database 116 stores electronic files of program data 120 which is used to generate an electronic program guide (or, "program guide"). Program data 120 includes characteristics for a TV show. These characteristics can be a title of a TV show, a story line of a TV show, a description of a TV show, production credits for a TV show, a critic's opinion of a TV show, a review for a TV show, a recommendation of a TV show, a program length range of a TV show, a commercial-free indicator for a TV show, a genre of a TV show, a rating for a TV show, a performer in a TV show, a director of a TV show, an award won by a TV show, a date relevant to a TV show, and other characteristics that represent facts specific to a TV show. Program data 120 also includes station identifiers, channel identifiers, schedule information, and so on. The terms program data, EPG data, and data of an electronic programming guide are used interchangeably throughout this discussion. For discussion purposes, an electronic file maintains program data 120 that may include, among other data, a program title 122, a program day or days 124 to identify which days of the week the program will be shown, and a start time or times 126 to identify the time that the program will be shown on the particular day or days of the week. The electronic file also maintains characteristics for each program as described above.

The EPG server 118 processes the EPG data prior to distribution to generate a published version of the program data which contains programming information for all channels for one or more days. The processing may involve any number of techniques to reduce, modify, or enhance the EPG data. Such processes might include selection of content, content compression, format modification, and the like. The EPG server 118 controls distribution of the published version of the program data from program data provider 104 to the content distribution system 106 using, for example, a file transfer protocol (FTP) over a TCP/IP network (e.g., Internet, UNIX, etc.). Further, the published version of the program data can be transmitted from the program data provider 104 via a satellite directly to the client device 108 by use of a satellite dish 134. The EPG data need not received via a video signal. Rather, the EPG data can be received by the client device 108 by tuning to a low bandwidth carrier signal that piggybacks with other signals and is generally transmitted at a lower data rate than video signals that are transmitted by satellite.

Content distribution system 106 includes a broadcast transmitter 128, one or more content processors 130, and one or more program data processors 132. Broadcast transmitter 128 broadcasts signals, such as cable television signals, across broadcast network 110. Broadcast network 110 can include a cable television network, RF, microwave, satellite, and/or data network, such as the Internet, and may also include wired or wireless media using any broadcast format or broadcast protocol. Additionally, broadcast network 110 can be any type of network, using any type of network topology and any network communication protocol, and can be represented or otherwise implemented as a combination of two or more networks.

Content processor 130 processes the content received from content provider 102 prior to transmitting the content across broadcast network 110. Similarly, program data processor 132 processes the program data received from program data provider 104 prior to transmitting the program data across broadcast network 110. A particular content processor 130 may encode, or otherwise process, the received content into a format that is understood by the multiple client devices 108(1), 108(2), ..., 108(N) coupled to broadcast network 110. Although Fig. 6 shows a single content provider 102, a single program data provider 104, and a single content distribution system 106, exemplary environment 100 can include any number of content providers and/or program data providers coupled to any number of content distribution systems.

Content distribution system 106 is representative of a headend service that provides EPG data, as well as content, to multiple subscribers. Each content distribution system 106 may receive a slightly different version of the program data that takes into account different programming preferences and lineups. The EPG server 118 creates different versions of EPG data (e.g., different versions of a program guide) that include those channels of relevance to respective headend services, and the content distribution system 106 transmits the EPG data to the multiple client devices 108(1), 108(2), ..., 108(N). In one implementation, for example, content distribution system 106 utilizes a carousel file system to repeatedly broadcast the EPG data over an out-of-band (OOB) channel to the client devices 108.

Client devices 108 can be implemented in a number of ways. For example, a client device 108(1) receives broadcast content from a satellite-based transmitter via satellite dish 134. Client device 108(1) is also referred to as a set-top box or a satellite receiving device. Client device 108(1) is coupled to television 136(1) for presenting the content received by the client device (e.g., audio data and video data), as well as a graphical user interface. A particular client device 108 can be coupled to any number of televisions 136 and/or similar devices that can be implemented to display or otherwise render content. Similarly, any number of client devices 108 can be coupled to a single TV 136. Each television 136 displays an enhanced EPG 170 on the screen thereof.

Client device 108(2) is also coupled to receive broadcast content from broadcast network 110 and provide the received content to associated television 136(2). Client device 108(N) is an example of a combination television 138 and integrated set-top box 140. In this example, the various components and functionality of the set-top box are incorporated into the television, rather than using two separate devices. The set-top box incorporated into the television may receive broadcast signals via a satellite dish (similar to satellite dish 134) and/or via broadcast network 110. In alternate implementations, client devices 108 may receive broadcast signals via a two-way network 109, such as the Internet, or any other broadcast medium.

Each client device 108 can execute a universal TV program listing and selection application as enhanced, modified, and improved by the present disclosure (the "enhanced EPG application"). The enhanced EPG application utilizes the TV program data that can be preloaded into the client device, received by broadcast via broadcast network 110 such as from content distribution system 106, or received from two-way network 109. When client device 108 executes the enhanced EPG application, a television viewer can locate one or more television show in a universe of all television shows that can be broadcast. Thus, the television viewer can select those TV programs that the viewer is interested in viewing and/or recording when ever they are broadcast on broadcast network 110.

The Enhanced application utilizes the program data to enable a television viewer to navigate through an onscreen program guide and locate television shows of interest to the viewer. With the enhanced EPG application, the television viewer can search and look at schedules of current and future programming, set reminders for upcoming programs, and/or enter instructions to record one or more television shows.

Also included in environment 100 are one or more network devices, such as a messaging server 150, that communicate with content distribution system 106 and with client devices 108 (1-N) through interconnected network 109, such as the Intemet. Interconnected network 109 allows two-way communication between client devices 108 (1-N) to messaging server 150. This communication allows client devices 108 (1-N) and/or messaging server 150 to transmit addressed messages over interconnected network 109. Each message can contain a message that is addressed to network resource, such as to an email address at an email server, to a Web site address of a web site on the Internet, to facsimile telephone number of a facsimile machine on a telephone network, or as is conventional with other message delivery modalities. By way of example, and not by way of limitation, a viewer can use the client device 108, or a personal computer or other network device to log on to network 109, such as the Internet, and to communicate with messaging server 150 or other network resource so as to locate any message that has been sent.

The messaging server 150 can be a network service, such as a Web hosting service, that stores data about any client device 108 or its respective viewer. The viewer can keep data at messaging server 150 that can in turn be accessed by other Web hosting services on the Internet where the viewer has permitted such access, which may be of a limited nature. An example of such a data provider is the Microsoft Network (MSN) of the Microsoft Corporation of Redmond, Washington. MSN provides a .NET™ PASSPORT® service that, among other services, stores data that can be retrieved by other Web services on the Intemet that are compatible with the .NET™ PASSPORT® service. In this example, the viewer can submit various contact information to messaging server 150. These contact information can be one or more addresses to which messages are to be sent.

Optionally, one or more of the program data providers 104 can include stored on-demand content, such as Video On-Demand (VOD) movie content. The stored on-demand content can be viewed with a client device 108 through an onscreen movie guide, for example, and a viewer can enter instructions to stream a particular movie, or other stored content, down to a corresponding client device 108.

### Exemplary Client Device

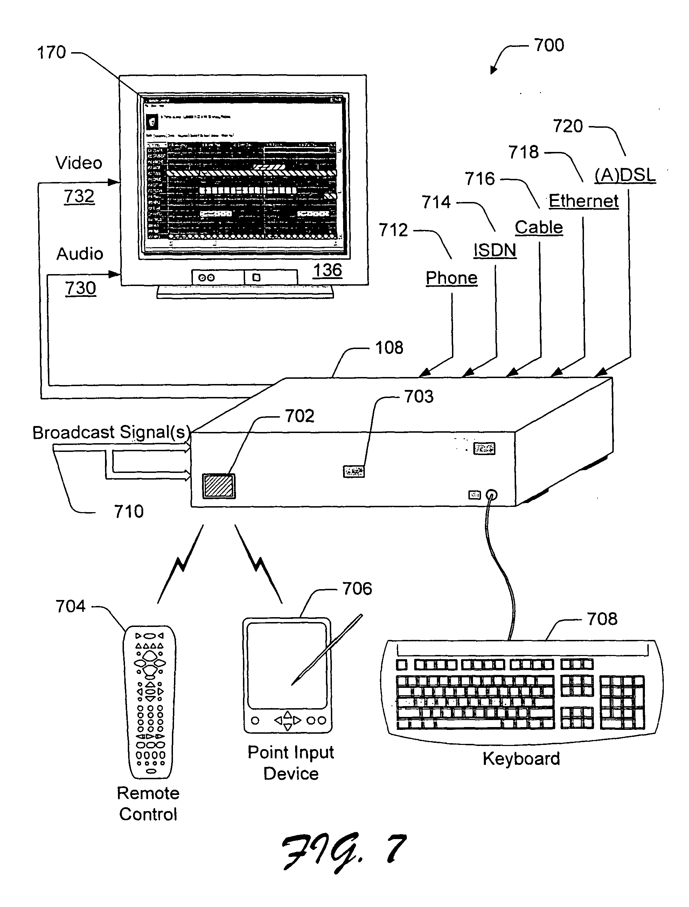

Fig. 7 illustrates an exemplary implementation 700 of a client device 108 shown as a standalone unit that connects to a television 136 that displays an enhanced EPG 170 on a screen. Client device 108 can be implemented in any number of embodiments, including as a set-top box, a satellite receiver, a TV recorder with a hard disk, a game console, an information appliance, and so forth. Client device 108 includes a wireless receiving port 702, such as an infrared (IR) or Bluetooth wireless port, for receiving wireless communications from a remote control device 704, a handheld input device 706, or any other wireless device, such as a wireless keyboard. Handheld input device 706 can be a personal digital assistant (PDA), handheld computer, wireless phone, or the like. Additionally, a wired keyboard 708 can be coupled to communicate with the client device 108. In alternate embodiments, remote control device 704, handheld device 706, and/or keyboard 708 may use an RF communication link or other mode of transmission to communicate with client device 108.

Client device 108 receives one or more broadcast signals 710 through from one or more broadcast sources, such as from a satellite or from a broadcast network. Client device 108 includes hardware and/or software for receiving and decoding broadcast signal 710, such as an NTSC, PAL, SECAM or other TV system video signal. Client device 108 also includes hardware and/or software for providing the viewer with a graphical user interface by which the viewer can, for example, access various network services, and perform other functions.

Client device 108 is capable of communicating through interconnected network 109 seen in Fig. 6 with other devices via one or more connections including a conventional telephone link 712, an ISDN link 714, a cable link 716, an Ethernet link 718, an ADSL and/or DSL link 720, and the like. Client device 108 may use any one or more of the various communication links 712-720 at a particular instant to communicate with any number of other devices and/or to establish a two-way communication with one or more network resources via network 109 seen in Fig. 6.

Client device 108 generates video signal(s) 720 and audio signal(s) 722, both of which are communicated to television 136. The video signals and audio signals can be communicated from client device 108 to television 136 via an RF (radio frequency) link, S-video link, composite video link, component video link, or other communication link. At reference numeral 703 in Fig. 7, client device 108 includes one or more lights or other request IDs identifying the current status of the device or for diagnostic reports to a viewer. Additionally, the client device may include one or more control buttons, switches, or other selectable controls for controlling operation of the device. A diagnostic visual and/or audible alarm device or mechanism at reference numeral 703 can emit a diagnostic representative of a system message so that the viewer may be warned visually and/or audibly.

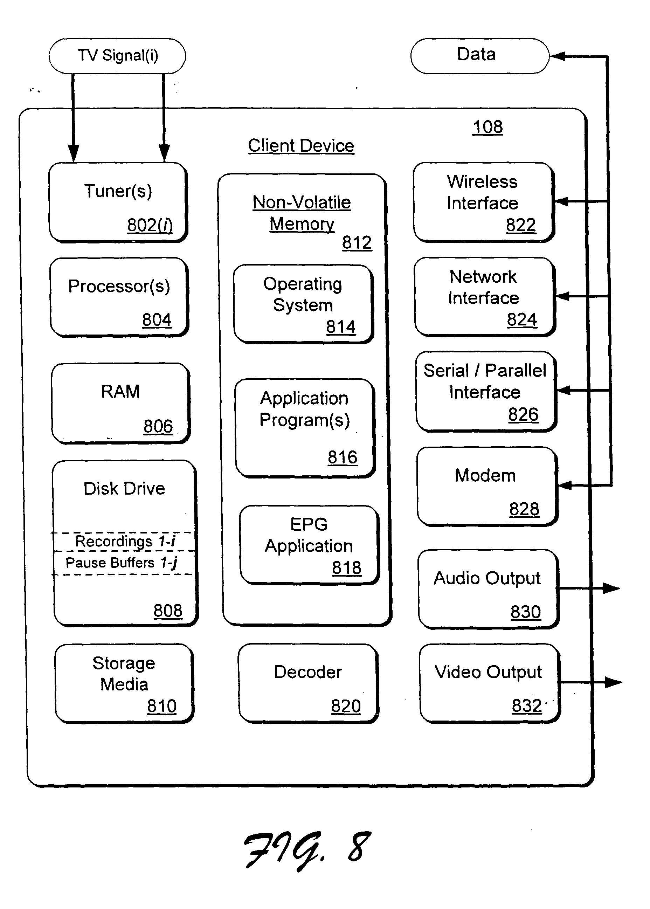

Fig. 8 illustrates selected components of client device(s) 108 shown in Figs. 1, 4, 6, and 7. Client device 108 includes one or more tuners 800(*i*). Tuners 800(*i*) are representative of one or more in-band tuners that tune to various frequencies or channels to receive television signals, as well as an out-of-band tuner that tunes to the broadcast channel over which the EPG data is broadcast to client device 108.

Client device 108 also includes one or more processors 804 and one or more memory components. Examples of possible memory components include a random access memory (RAM) 806, a disk drive 808, a mass storage component 810 such as a tape in a tape drive or removable media component in a removable media drive, and a non-volatile memory 812 (e.g., ROM, Flash, EPROM, EEPROM, etc.). Disk drive 808 can have one or a plurality of audiovisual recordings (*i*) and one or a plurality of pause buffers (*j*) stored thereon. A TV program database can be stored on disk drive 808 to keep respective TV program characteristics that are communicated to the client device 108 in a broadcast EPG The TV program database can also keep the viewer selections entered at one or more questionnaires, for example as seen in Figs. 2-3. Recordings (*i*), pause buffers (*j*), and the TV program database can also be stored in one or more other memory devices at client device 108, such as in non-volatile memory 812, RAM 806, and/or storage media 810. Alternatively, recordings (*i*), pause buffers (*j*), and the TV program database can also be stored remote from client device 108 at a network resource in communication with client device 108 through interconnected network 109 seen in Fig. 6.

Alternative implementations of client device 108 can include a range of processing and memory capabilities, and may include more or fewer types of memory components than those illustrated in Fig. 8. For example, full-resource clients can be implemented with substantial memory and processing resources, including a disk drive 808 to store content for replay by the viewer. Low-resource clients, however, may have limited processing and memory capabilities, such as a limited amount of RAM 806, no disk drive 808, and limited processing capabilities. Nevertheless it is intended that client device 108 include a capability for video recording, either locally or remotely from client device 108.

Processor(s) 804 process various instructions to control the operation of client device 108 and to communicate with other electronic and computing devices. The memory components (e.g., RAM 806, disk drive 808, storage media 810, and non-volatile memory 812) store various information and/or data such as content, EPG data, configuration information for client device 108, and/or graphical user interface information.

An operating system 814 and one or more application programs 816 may be stored in non-volatile memory 812 and executed on processor 804 to provide a runtime environment. A runtime environment facilitates extensibility of client device 108 by allowing various interfaces to be defined that, in turn, allow application programs 816 to interact with client device 108. In the illustrated example, an enhanced EPG application 818 and variations thereof, is stored in memory 812 to operate on the data of an electronic programming guide and generate various embodiments of the enhanced EPG display disclosed herein.

Reports can be output from client device 108, as well as other communications, which can be communicated in a one or two-way communication through interconnected network 109 see in Fig. 6. These communications can be made with various network resources using network interface 824, wireless interface 822, serial/parallel interface 826, modem 828, or other well known communication hardware/software algorithms and protocol for computing devices.

The application programs 816 that may be implemented at client device 108 include a browser to browse the Web so as to view diagnostics at a Web site, an electronic mail (email) program to facilitate email to transmit message emails to an email address, a facsimile transmission program to initiate a facsimile transmission to a facsimile machine through phone line 712 seen in Fig. 7 so as to send a facsimile message with respect to client device 108, a Short Message Service (SMS) transmission program to initiate a transmission of a text message with respect to client device 108 to a mobile pager on a page channel, and so on.

Client device 108 can also include other components pertaining to a television entertainment system which are not illustrated in this example for simplicity purposes. For instance, client device 108 can include a user interface application and user interface lights, buttons, controls, etc. to facilitate viewer interaction with the device.

Client device 108 also includes a decoder 820 to decode a broadcast video signal, such as an NTSC, PAL, SECAM or other TV system video signal. Alternatively, a decoder for client device 108 can be implemented, in whole or in part, as a software application executed by processor(s) 804. Wireless interface 822 allows client device 108 to receive input commands and other information from a viewer-operated input device, such as from a remote control device or from another IR, Bluetooth, or similar RF input device.

Network interface 824 and serial and/or parallel interface 826 allows client device 108 to interact and communicate with other electronic and computing devices via various communication links. Although not shown, client device 108 may also include other types of data communication interfaces to communicate with other devices. Modem 828 facilitates client device 108 communications with other electronic and computing devices via a conventional telephone line. Components seen at reference numerals 816 and 822-828 facilitate applications where client device 108 has Internet access or communicates data on a two-way network.

Client device 108 also includes an audio output 830 and a video output 832 that provide signals to a television or other device that processes and/or presents or otherwise renders the audio and video data. Although shown separately, some of the components of client device 108 may be implemented in an application specific integrated circuit (ASIC). Additionally, a system bus (not shown) typically connects the various components within client device 108. A system bus can be implemented as one or more of any of several types of bus structures, including a memory bus or memory controller, a peripheral bus, an accelerated graphics port, or a local bus using any of a variety of bus architectures. By way of example, such architectures can include an Industry Standard Architecture (ISA) bus, a Micro Channel Architecture (MCA) bus, an Enhanced ISA (EISA) bus, a Video Electronics Standards Association (VESA) local bus, and a Peripheral Component Interconnects (PCI) bus also known as a Mezzanine bus.

General reference is been made herein to one or more client devices, such as client device 108. As used herein, "client device" means any electronic device having data communications, data storage capabilities, and/or functions to process signals, such as broadcast signals, received from any of a number of different sources.

Client device 108 can include a variety of computer readable media identified as communication media. Communication media typically embodies computer readable instructions, data structures, program modules, or other data in a modulated data signal such as a carrier wave or other transport mechanism and includes any information delivery media.

The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared, and other wireless media. Combinations of any of the above are also included within the scope of computer readable media.

In a networked environment, such as that illustrated with computing environment 100 seen in Fig. 6, program modules or portions thereof, may be stored in a remote memory storage device. By way of example, application programs 816 and enhanced EPG application 818 may reside on a memory device of a remote computer and/or server. For purposes of illustration, Fig. 8 shows the application programs 816 and enhanced EGP application 818 in illustrated discrete blocks, although it is recognized that such programs and components reside at various times in different storage components of the client device 108, and are executed by the processor(s) 804 of the client device 108.

Although the invention has been described in language specific to structural features and/or methodological steps, it is to be understood that the invention defined in the appended claims is not necessarily limited to the specific features or steps described. Rather, the specific features and steps are disclosed as preferred forms of implementing the claimed invention.

## Claims

1. A method comprising:
displaying one or more viewing interests and a highlighting corresponding thereto;
receiving a selection of one or more said viewing interests; and
displaying an Electronic Programming Guide (EPG) within a programming range, wherein:
the EPG includes one or more TV shows for one or more channels in one or more time slots;
each said TV show has a plurality of characteristics; and
each said TV show in the programming range for which the characteristics thereof match the selection of one or more of said viewing interests is displayed with the corresponding highlighting.

2. The method as defined in Claim 1, wherein each said TV show in the programming range for which the characteristics thereof do not match the selection of one or more of said viewing interests is displayed without any said highlighting.

3. A method as defined in Claim 1, wherein each said viewing interest and each said characteristic of each said TV show is selected from a group consisting of:
a character string in metadata comprising the characteristics of a TV show;
an alphanumeric text string in a title of a TV show;
an alphanumeric text string in a story line of a TV show;
an alphanumeric string in a description of a TV show;
an alphanumeric string in production credits of a TV show;
an alphanumeric text string in a critic's opinion of a TV show;
an alphanumeric text string in a review of a TV show;
an alphanumeric text string in a recommendation of A TV show;
a program length range of A TV show;
a commercial-free indicator of a TV show;
a genre of a TV show;
a rating range of a TV show;
a performer in a TV show;
a director of a TV show;
an award won by a TV show; and
a date range of a TV show.

4. The method as defined in Claim 1, further comprising retrieving data for the EPG, wherein:
the data includes the plurality of said TV shows for a plurality of said time slots; and
a plurality of said characteristics respectively corresponding to the plurality of said TV shows.

5. The method as defined in Claim 1, wherein the programming range comprises:
a predetermined range of days;
a predetermined time period with each said day; and
a predetermined set of the channels.

6. The method as defined in Claim 1, wherein the highlighting is selected from the group consisting of a text appearance, a border appearance, a background appearance, shading within a border, a text color, an alignment of text, a border appearance, a background color, a shading, an icon, a typeface, a font, a font style, a font size, a text effect, and combinations thereof.

7. The method as defined in Claim 1, further comprising receiving, from the display of the EPG, a selection of one said TV show in the programming range.

8. The method as defined in Claim 7, further comprising tuning to the channel of the selected one said TV show in the programming range.

9. The method as defined in Claim 1, further comprising:
receiving, from the display of the EPG, a selection of one said TV show in the programming range;
receiving a command to match the characteristics of the selected one said TV show in the programming range against the characteristics of other said TV shows in the EPG; and
displaying the EPG within the programming range such that:
each said TV show in the programming range that has characteristics that match the characteristics of the selection of the one said TV show in the programming range is displayed with a predetermined highlighting; and
each said TV show in the programming range that has characteristics that does not match the characteristics of the selection of the one said TV show in the programming range is displayed without the predetermined highlighting.

10. The method as defined in Claim 1, wherein the displaying an EPG within a programming range further comprises displaying one or more controls for selecting the one said TV show in the programming range for which the characteristics thereof are to be matched against the characteristics of other said TV shows in the EPG.

11. The method as defined in Claim 1, further comprising identifying the characteristics of each said TV show in the EPG within the programming range that match the received selections of the one or more said viewing interests.

12. The method as defined in Claim 1, wherein when there are a plurality of said matches for one said TV show in the programming range, then prioritizing the selected said viewing interests to determine the corresponding highlighting.

13. A computer readable media comprising instructions that, when executed by one or more processors, performs the method of Claim 1.

14. An EPG display comprising a plurality of TV shows for one or more channels in one or more time slots that in a programming range; wherein:
each said TV show has a plurality of characteristics; and
each said TV show in the programming range having the characteristics thereof that match one or more viewing interests previously selected by a TV viewer is displayed with a visual cue corresponding to the selected one or more viewing interests.

15. The EPG as defined in Claim 14, wherein each said TV show in the programming range for which the characteristics thereof do not match the selected one or more viewing interests is not displayed with the visual cue corresponding to the selected one or more viewing interests.

16. The EPG display as defined in Claim 14, wherein each said viewing interest and each said characteristic of each said TV show is selected from a group consisting of:
a character string in the characteristics of a TV show;
a character string in a title of a TV show;
a character string in a description of a TV show;
a character string in production credits of a TV show;
a genre of a TV show;
a rating range of a TV show;
a performer in a TV show;
a director of a TV show;
an award won by a TV show; and
a date range of a TV show.

17. The EPG display as defined in Claim 14, wherein the programming range comprises:
a predetermined range of days;
a predetermined time period with each said day; and
a predetermined set of the channels.

18. The EPG display as defined in Claim 14, wherein the visual cue is selected from the group consisting of a text appearance, a border appearance, a background appearance, shading within a border, a text color, a border appearance, a background color, a shading, an icon, a typeface, a font, a font style, a font size, a text effect, and combinations thereof.

19. The EPG display as defined in Claim 14, wherein when there are a plurality of said matches for one said TV show in the programming range, then prioritizing the selected said viewing interests to determine the visual cue corresponding to the selected one or more viewing interests.

20. The EPG display as defined in Claim 14, further comprising one or more controls for use by the TV viewer to select the viewing interests, wherein each said control is selected from the group consisting of:
one or more pull down menus that, when activated, display the viewing interests and selections for the respective highlighting corresponding thereto;
one or more pull down menus that, when activated, display a category of the viewing interests, a field for receiving input of a character string, and the highlighting corresponding to the category and the character string; and
a combination of the foregoing.

21. A user interface (UI) comprising:
a first panel depicted on a first display screen to depict one or more controls for selecting one or more viewing interests and a highlighting corresponding thereto; and
a second panel depicted on a second display screen to depict an EPG including one or more TV shows, each having one or more characteristics, for one or more channels in one or more time slots within a programming range, and also including: the TV shows in the programming range have characteristics that match a selection of one or more of said viewing interests received from the first display screen such that the TV shows in the first portion are displayed on the EPG with the corresponding highlighting.

22. The UI as defined in Claim 21, wherein the second panel further comprises one or more TV shows in the programming range that have characteristics that do not match the selection of one or more said viewing interests received from the first display screen such that the TV shows in the second portion are displayed on the EPG without any selected said highlighting.

23. The UI as defined in Claim 21, wherein each said viewing interest and each said characteristic of each said TV show is selected from a group consisting of:
a character string in the characteristics of a TV show;
a character string in a title of a TV show;
a character string in a description of a TV show;
a character string in production credits of a TV show;
a genre of a TV show;
a rating range of a TV show;
a performer in a TV show;
a director of a TV show;
an award won by a TV show; and
a date range of a TV show.

24. The UI as defined in Claim 21, wherein the programming range comprises:
a predetermined range of days;
a predetermined time period with each said day; and
a predetermined set of the channels.

25. The UI as defined in Claim 21, wherein the highlighting is selected from the group consisting of a text appearance, a border appearance, a background appearance, shading within a border, a text color, a border appearance, a background color, a shading, an icon, a typeface, a font, a font style, a font size, a text effect, and combinations thereof.

26. The UI as defined in Claim 21, wherein each said control is selected from the group consisting of:
one or more pull down menus that, when activated, display the selectable viewing interests and the respective highlighting corresponding thereto;
one or more pull down menus that, when activated, display a category of the selectable viewing interests, a field for receiving input of a character string, and selections for the highlighting corresponding to the category and the character string; and
a combination of the foregoing.

27. An apparatus comprising:
means for receiving data representing one or more TV shows for one or more channels in one or more time slots, wherein each said TV show has a plurality of characteristics;
means for outputting a display of one or more viewing interests and a highlighting corresponding thereto;
means for receiving a selection of one or more said viewing interests and the respective highlighting corresponding thereto;
means for determining each said TV show in a programming range for which the characteristics thereof match the selection of one or more of said viewing interests; and
means for outputting a display of an EPG within the programming range, wherein each said TV show corresponding to each said match is displayed with the highlighting corresponding to the match.

28. The apparatus as defined in Claim 27, further comprising:
means for receiving a selection of one said TV show displayed on the EPG; and
means for tuning to the channel of the selected one said TV show.

29. The apparatus as defined in Claim 27, further comprising:
means for receiving a selection of one said TV show displayed on the EPG;
means for receiving a command to match the characteristics of the selected of one said TV show against the characteristics of other said TV shows in the EPG; and
means for displaying the EPG within the programming range such that:
each said TV show in the programming range that has characteristics that match the characteristics of the selection of the one said TV show in the programming range is displayed with a predetermined highlighting; and
each said TV show in the programming range that has characteristics that does not match the characteristics of the selection of the one said TV show in the programming range is displayed without the predetermined highlighting.

30. The apparatus as defined in Claim 27, wherein the means for receiving a code segment that, when executed by a client device, provide a UI selected from the group consisting of:
one or more pull down menus that, when activated, display the viewing interests and selections for the respective highlighting corresponding thereto;
one or more pull down menus that, when activated, display a category of the viewing interests, a field for receiving input of a character string, and selections for the highlighting corresponding to the category and the character string; and
a combination of the foregoing.

31. The apparatus as defined in Claim 27, wherein each said viewing interest and each said characteristic of each said TV show is selected from a group consisting of:
a character string in metadata comprising the characteristics of a TV show; an alphanumeric text string in a title of a TV show;
an alphanumeric text string in a story line of a TV show;
an alphanumeric string in a description of a TV show;
an alphanumeric string in production credits of a TV show;
an alphanumeric text string in a critic's opinion of a TV show;
an alphanumeric text string in a review of a TV show;
an alphanumeric text string in a recommendation of A TV show;
a program length range of A TV show;
a commercial-free indicator of a TV show;
a genre of a TV show;
a rating range of a TV show;
a performer in a TV show;
a director of a TV show;
an award won by a TV show; and
a date range of a TV show.

32. The apparatus as defined in Claim 27, wherein the programming range comprises:
a predetermined range of days;
a predetermined time period with each said day; and
a predetermined set of the channels.

33. The apparatus as defined in Claim 27, wherein the highlighting is selected from the group consisting of a text appearance, a border appearance, a background appearance, shading within a border, a text color, an alignment of text, a border appearance, a background color, a shading, an icon, a typeface, a font, a font style, a font size, a text effect, and combinations thereof.

34. The apparatus as defined in Claim 27, wherein the means for receiving a selection of one or more said viewing interests and the respective highlighting corresponding thereto further comprises means for displaying one or more controls by which each said selection is made.

35. The apparatus as defined in Claim 27, wherein when there is a plurality of said matches for one said TV show in the programming range, then prioritizing the selected said viewing interests to determine the highlighting corresponding to one said match of the plurality of said matches.

36. One or more computer-readable media comprising:
a first code segment that, when executed, provides a UI by which a TV viewer selects viewing interests and highlighting corresponding thereto; and
a second code segment that, when executed, provides an EPG display using EPG data that includes a plurality of TV shows in a programming range each having one or more characteristics, wherein TV shows in the EPG display having characteristics that match the selected viewing interests are displayed with the highlighting corresponding thereto.

37. The computer readable medium as defined in Claim 36, wherein each said TV show in the programming range for which the characteristics thereof do not match the selection of one or more of said viewing interests is displayed without any said highlighting.

38. The computer readable medium as defined in Claim 36, wherein each said viewing interest and each said characteristic of each said TV show is selected from a group consisting of:
a character string in metadata comprising the characteristics of a TV show; an alphanumeric text string in a title of a TV show;
an alphanumeric text string in a story line of a TV show;
an alphanumeric string in a description of a TV show;
an alphanumeric string in production credits of a TV show;
an alphanumeric text string in a critic's opinion of a TV show;
an alphanumeric text string in a review of a TV show;
an alphanumeric text string in a recommendation of A TV show;
a program length range of A TV show;
a commercial-free indicator of a TV show;
a genre of a TV show;
a rating range of a TV show;
a performer in a TV show;
a director of a TV show;
an award won by a TV show; and
a date range of a TV show.

39. The computer readable medium as defined in Claim 36, further comprising a third code segment that, when executed, retrieves data for the EPG display within the, wherein:
the data includes a plurality of said TV shows for a plurality of time slots; and
a plurality of said characteristics respectively corresponding to the TV shows.

40. The computer readable medium as defined in Claim 36, wherein the programming range comprising:
a predetermined range of days;
a predetermined time period with each said day; and
a predetermined set of the channels.

41. The computer readable medium as defined in Claim 36, wherein the highlighting is selected from the group consisting of a text appearance, a border appearance, a background appearance, shading within a border, a text color, an alignment of text, a border appearance, a background color, a shading, an icon, a typeface, a font, a font style, a font size, a text effect, and combinations thereof.

42. The computer readable medium as defined in Claim 36, further comprising a third code segment that, when executed by a client device, the client device receives from the EPG display a selection of one said TV show in the programming range.

43. The computer readable medium as defined in Claim 42, further comprising a fourth code segment that, when executed by the client device, the client device tunes to the channel of the selected one said TV show in the programming range.

44. The computer readable medium as defined in Claim 36, wherein when:
the second code segment is executed; and
there is more than one said match for one said TV show, the selected said viewing interests of the matches are prioritized to determine the corresponding highlighting.
